# FASCICULE DE BREVET EUROPEEN

(11) **EP 2 861 634 B1**
(45) Date de publication et mention de la délivrance du brevet: **05.08.2020**
(21) Numéro de dépôt: 13747510.9
(22) Date de dépôt: 14.06.2013
(51) Int. Cl.: C08F 6/00, C08F 6/06, A61F 2/14, C08L 33/20

(54) **PROCÉDÉ DE PRÉPARATION D'OBJETS EN HYDROGEL BIOCOMPATIBLE POUR LEURS APPLICATIONS DANS LE DOMAINE MÉDICAL, ET PLUS PARTICULIÈREMENT EN OPHTALMOLOGIE**
VERFAHREN ZUR HERSTELLUNG VON GEGENSTÄNDEN AUS BIOKOMPATIBLEM HYDROGEL FÜR VERWENDUNGEN IN DER MEDIZIN, INSBESONDERE IN DER OPHTHALMOLOGIE
PROCESS FOR PREPARING OBJECTS MADE OF BIOCOMPATIBLE HYDROGEL FOR USES THEREOF IN THE MEDICAL FIELD, AND MORE PARTICULARLY IN OPHTHALMOLOGY

(30) Priorité: 15.06.2012 FR 1255631
(43) Date de publication de la demande: 22.04.2015
(73) Titulaire: Laroche, Laurent, 75006 Paris (FR)
(72) Inventeur: Laroche, Laurent, 75006 Paris (FR)
(74) Mandataire: Gevers & Orès
(86) Numéro de dépôt international: PCT/IB2013/054874
(87) Numéro de publication internationale: WO 2013/186747

(56) Documents cités:
- EP-A1- 0 688 569
- FR-A1- 2 630 638
- FR-A1- 2 810 552
- DATABASE COMPENDEX [Online] ENGINEERING INFORMATION, INC., NEW YORK, NY, US; 15 décembre 1997 (1997-12-15), HONIGER J ET AL: "New anionic polyelectrolyte hydrogel for corneal surgery", XP002715501, Database accession no. EIX98083993684 -& JOURNAL OF BIOMEDICAL MATERIALS RESEARCH, vol. 37, no. 4, 15 décembre 1997 (1997-12-15), pages 548-553, XP002715980, JOHN WILEY & SONS INC US DOI: 10.1002/(SICI)1097-4636(19971215)37:4<548: :AID-JBM14>3.0.CO;2-4

## Description

La présente invention concerne un procédé de fabrication d'un objet en hydrogel biocompatible par moulage d'une solution polymérique dans un moule réalisé en un matériau particulier. L'invention concerne aussi les objets en hydrogel biocompatibles issus de ce procédé tels que, par exemple, des lentilles (ou lenticules) intra-cornéennes implantables dans la cornée ou tous autres implants utilisables en ophtalmologie.

Plusieurs procédés de fabrication d'objets en hydrogel à usage médical et/ou chirurgical obtenus par moulage d'une solution polymérique sur un moule de forme adaptée ont déjà été décrits dans l'art antérieur. Parmi ceux-ci, on peut citer la méthode de « spin casting », aussi appelée centrifugation, utilisée par Wichterle (1960) pour la fabrication de lentilles de contact en hydrogel obtenus à partir d'hydroxyéthylméthacrylate (HEMA) catalysées, et dont le temps de gélification dépend entre autre de la température. Une autre méthode de réalisation d'objets en hydrogels consiste en la gélification par refroidissement d'une solution polymérique à une température inférieure à la température du point de gélification (Brevet FR 2 051 147). Le principal avantage de ce procédé est la compensation de variation volumique de la solution polymérique placée à l'intérieur du moule. Ce procédé, qui est basé sur la gélification par abaissement de la température en dessous du point de gélification, ne permet pas à une solution copolymérique anionique d'AN69 (copolymère fourni par la Société GAMBRO) l'obtention d'hydrogels transparents, mais conduit au contraire à des hydrogels translucides, non utilisables en chirurgie ophtalmologique réfractive.

D'autres objets en hydrogels peuvent être réalisés par découpe mécanique dans un bloc d'hydrogel à température ambiante, en utilisant les lames, des micro-scies ou des lasers, ou par découpe par micro-jet d'eau à très forte pression, ou par cryo-usinage à très basse température. Cette méthode a également été testée pour la fabrication de lentilles en hydrogel à base de copolymère d'AN-69, mais sans succès.

Le copolymère AN-69 est utilisé depuis de nombreuses années pour la fabrication de membranes d'hémodialyse (J. Denis et al., Gut, 1978, 19, 787-793). L'hydrogel issu de ce copolymère a également été utilisé pour l'encapsulation des îlots de Langerhans dans le développement du pancréas bio-artificiel (J. Honiger et al., The International Journal of Artificial Organs, 1994, 17, 046-052), ou encore pour l'encapsulation des hépatocytes en vue de développer un foie bio-artificiel (J. Honinger et al., Biomaterials, 1995, 16, 753-759 ; R. Sarkis et al., Transplantation, 2000, Vol. 70, 58-64 ; Journal of Hepatology, 2001, 35, 208-216 ; E. Baldini et al., Transplantation Proceeding, 2009, 41(4), 1367-1369). Dans des études de thérapie de la polyarthrite, des cellules murines ont également été encapsulées dans une fibre creuse en hydrogel d'AN-69 (N. Bessis et al., Clin. Exp. Immunol., 1999, 117, 376-382), et dans les capsules réalisées avec ce même hydrogel (N. Bessis et al., Rhumatologie, 2003, 70, 855-7). Des cellules produisant de l'érythropoïétine ont aussi été encapsulées dans des fibres creuses en hydrogel d'AN-69 (E. Payen et al., Haematologica, 1999, 84, EHA-4). Aussi, le copolymère AN-69 a déjà largement démontré ses propriétés de biocompatibilité et d'hémocompatibilité, et ses capacités à ne pas activer le système du complément (J. Honinger et al., J. Biomed. Mater. Res., 1997, 37, 548-553). L'hydrogel issu de ce copolymère est également largement utilisé dans le domaine de l'ophtalmologie. Des lentilles intra-cornéennes ont en effet déjà été réalisées à partir de ce copolymère (Brevet EP 0 347 267 ; L. Laroche et al., Macromol. Symp., 1995, 100, 51-55) et implantées sur des animaux, puis évalués cliniquement chez l'homme depuis 20 ans déjà. Le même hydrogel d'AN-69 a aussi été utilisé pour l'étude de la prolifération des cellules épithéliales pour le développement de lentilles d'épikératophakie (F. Maury et al., Journal of Materials Science-Materials in medicine, 1997, 8, 571-576).

Le thermoformage de pastilles d'hydrogel pour la réalisation de lentilles intra-cornéennes a été exploité depuis la fin des années 80 par J. Honiger et L. Laroche. La qualité optique de ces lentilles était parfaite. Avant l'implantation dans la cornée, les lentilles étaient décontaminées avec de l'acide peracétique, puis rincées avec du sérum physiologique stérile. Les nouvelles exigences de la Pharmacopée, devenue plus sévères, peuvent exiger une stérilisation, et non plus seulement une décontamination, des objets implantables. En les stérilisant par les méthodes prescrites telles que la chaleur humide, les rayons gamma, ou les électrons accélérés, les lentilles thermoformées peuvent changer de forme, et leur puissance optique s'en trouvée altérée.

Ainsi, le point faible des implants intra-cornéens issus des procédés décrits ci-dessus est leur capacité médiocre à conserver leur forme lors de l'étape de stérilisation.

La Demanderesse a ainsi cherché à fournir des hydrogels transparents etbiocompatibles, pour leur mise en œuvre pour la préparation d'objets à usage médical ou chirurgical hautement fiables, et en particulier pour des implants oculaires présentant une perméabilité appropriée à différentes molécules biologiques.

L'objet de la présente invention est défini par les revendications 1 à 14.

Ainsi, le premier objet de la description est un procédé de fabrication d'un objet en hydrogel biocompatible comprenant les étapes suivantes :
(i) la préparation d'une solution polymérique par dissolution d'un copolymère d'acrylonitrile et d'un co-monomère oléfiniquement insaturé porteur de groupes anioniques dans un solvant aprotique, éventuellement en présence d'un non solvant,
(ii) la mise en forme et le début de la gélification de la solution polymérique obtenue à l'issue de l'étape (i) dans un moule ayant la forme de l'objet souhaité, ledit moule étant constitué en un matériau contenant ledit non solvant ou en un matériau perméable audit non solvant, ladite étape étant de préférence réalisée à température ambiante,
(iii) l'immersion de l'objet en cours de gélification issu de l'étape (ii) dans un non solvant, pendant une durée suffisante pour permettre l'échange total du solvant par ledit non solvant, et obtenir l'objet en hydrogel.

De manière tout à fait inattendue, la Demanderesse a constaté que l'utilisation d'un moule réalisé en un matériau spécifique constitué de non solvant et perméable audit non solvant permettait à la fois d'assurer un échange entre le solvant présent au sein de la solution polymérique et le non solvant au moment de l'étape de gélification, et de former un hydrogel (dans le cas où le non solvant est de l'eau) ayant la forme désirée. En effet, c'est la présence du non solvant au sein du moule qui fait gélifier la solution polymérique moulée. Les implants oculaires et/ou intra-cornéens en hydrogel obtenus à l'issu du procédé faisant l'objet de la description présentent, outre leur caractère d'inertie vis-à-vis des cellules biologiques :
- d'excellentes propriétés optiques : transparence parfaite à la lumière visible, absorption des rayons UV, indice de réfraction voisin de celui de la cornée,
- de très bonnes propriétés physico-chimiques : haute perméabilité à l'eau, au sérum physiologique, aux petites et moyennes molécules, une perméabilité aux gaz dissous, une haute hydrophilie, une nature chimique dépourvue de groupements toxiques,
- une excellente stabilité dimensionnelle,
- des propriétés biologiques particulières : non-biorésorbable dans le milieu physiologique, stérilisable et/ou re-stérilisable, bonne résistance au vieillissement dans ce milieu, bonne tolérance tissulaire des sites d'implantation dans le stroma cornéen (sans provoquer d'altération de l'épithélium et de l'endothélium cornéens), faible affinité aux protéines.

La dissolution de l'étape (i) peut être réalisée, sous agitation, à une température allant de la température ambiante jusqu'à 70°C, et de préférence à une température d'environ 50°C.

L'étape (i) du procédé faisant l'objet de la description consiste en la préparation par dissolution d'un copolymère acrylonitrile/co-monomère présentant avantageusement un rapport molaire allant de 90/10 à 100/0, et de préférence allant de 95/5 à 99/1.

Selon un mode de réalisation avantageux, les groupes anioniques du co-monomère oléfiniquement insaturé sont choisis parmi les groupes sulfonate, carboxylate, phosphate, phosphonate et sulfate.

Le copolymère acrylonitrile/co-monomère est avantageusement un copolymère acrylonitrile-méthallylsulfonate de sodium tel que le copolymère AN-69 (fournisseur GAMBRO). Ces copolymères ne présentent pas d'interaction avec les cellules et ont donc une tolérance nettement améliorée.

Le solvant aprotique dans lequel le copolymère acrylate/co-monomère est dissous est avantageusement choisi parmi les solvants aprotiques polaires organiques ou inorganiques, et de préférence parmi le diméthylformamide (DMF), le diméthylsulfoxyde (DMSO), la N,N-diméthylacétamide (DMAC), la N-méthylpyrrolidone (NMP).

Le non solvant est choisi parmi l'eau, les solutions aqueuses de sel minéral et les solutions aqueuses de sel organique.

Selon une disposition avantageuse de ce mode de réalisation, lorsque le non solvant est une solution aqueuse de sel, ladite solution est à une concentration comprise entre 0,5 et 5% en poids, de manière à obtenir dans la solution polymérique une concentration en sel comprise entre 0,03 et 1% en poids, et de préférence entre 0,05 et 1%.

De manière encore plus préférée, les sels minéraux et organiques sont le chlorure de sodium (solution physiologique) ou de potassium, l'iodate de sodium ou de potassium, le bicarbonate de sodium ou de potassium, le chlorate de sodium ou de potassium, le périodate de sodium ou de potassium, le nitrate de sodium ou de potassium, le citrate de sodium ou de potassium, le tartrate de sodium ou de potassium, l'ascorbate de sodium ou de potassium, l'acétate de sodium ou de potassium, le lactate de sodium ou de potassium. La solution aqueuse de sel préférée est une solution de chlorure de sodium.

Selon un mode de réalisation avantageux, le moule de l'étape (ii) est un moule en hydrogel.

Au sens où l'entend la description, un hydrogel est un matériau constitué de chaînes de polymère ayant des sites hydrophiles. Le moule de l'étape (ii) peut être un moule en hydrogel à base d'agarose, d'alginates, de polyhydroxyéthylméthacrylate (PHEMA), de polyhydroxypropylméthacrylate (PHPMA) ou de polyacrylate (de sodium ou de potassium).

De manière avantageuse, le moule de l'étape (ii) est constitué :
- de 1 à 10% en poids, et de préférence de 2 à 6%, d'agarose ou d'alginates, et de
- de 90 à 99% en poids, et de préférence de 94 à 98%, d'eau ou d'une solution aqueuse de sel minéral ou d'une solution aqueuse de sel organique.

De manière particulièrement préférée, le moule de l'étape (ii) est un moule en hydrogel d'agarose.

Au cours de l'étape (ii) du procédé faisant l'objet de la description, également connue sous le terme d'étape de « démixtion » ou étape de « séparation de phase » de la solution polymérique homogène obtenue à l'issue de l'étape (i), un hydrogel se forme.

Selon le diagramme ternaire (copolymère/solvant/non solvant), la courbe d'équilibre sépare une zone où les trois composants sont miscibles d'une zone où les deux autres phases se forment (une phase solide riche en polymère, et une phase liquide pauvre ou appauvrie en polymère). Au cours de la formation de l'hydrogel, le système évolue depuis la solution polymérique initiale jusqu'à une composition où tout le solvant est remplacé par le non solvant, ceci afin de transformer le gel en hydrogel. Le passage de la forme liquide à la forme gélifiée est déclenché par contact de la solution polymérique avec le non solvant contenu dans le moule de forme préalablement choisie en fonction de l'application ultérieure visée. Les couches superficielles de la solution polymérique qui sont en contact direct avec la surface du moule contenant le non solvant, commencent à gélifier et prennent la forme du moule. Plus l'objet moulé a une épaisseur importante, plus le temps de gélification est long.

L'étape (iii) d'immersion de l'objet en cours de gélification peut être réalisée en deux temps :
- dans un premier temps : immersion de l'objet en cours de gélification dans un bain froid de non solvant, de préférence à une température allant de 0 à 10°C, pendant une durée pouvant aller de 5 à 15 minutes, et
- dans un deuxième temps : immersion de l'objet en cours de gélification dans un bain de non solvant à température ambiante, pendant une durée pouvant aller de 15 à 45 minutes, et de préférence pendant environ 30 minutes.

Après les étapes (i) à (iii), le procédé faisant l'objet de la description peut également comprendre une étape optionnelle de stérilisation. Avantageusement, cette étape de stérilisation est réalisée par radio-stérilisation, par exemple par rayons gamma ou par électrons accélérés, et de manière plus préférée par radio-stérilisation par rayons gamma ou par électrons accélérés.

La présente description concerne également les objets en hydrogel biocompatible obtenus selon le procédé faisant l'objet de la description.

Le terme d'objet en hydrogel biocompatible doit être compris comme un objet fabriqué en un matériau non vivant utilisé comme un dispositif médical destiné à interagir au contact des systèmes biologiques, sans les dénaturer, c'est-à-dire sans entraîner d'anomalies dans le comportement des tissus cellulaires et sans provoquer d'intoxication des liquides biologiques circulant dans les viscères du corps humain ou animal. Ce contact qui est évident dans le cas d'un implant, doit être étendu aux contacts qui se réalisent à la surface ou à l'extérieur du corps humain ou animal, comme par exemple ceux qui se produisent avec le sang dans le cas d'une hémodialyse ou avec la cornée dans le cas de lentilles de contact.

Ces objets peuvent être des films, fils, joncs ou implants à usage médical, biologique, ophtalmologique et/ou extra-ophtalmologique.

Selon un autre mode de réalisation avantageux, les objets en hydrogel biocompatible faisant l'objet de la description sont des implants oculaires. Il peut s'agir de lentilles ayant ou non une puissance optique ou réfractive, et plus particulièrement des lentilles intra-cornéennes destinées à être implantées dans la cornée pour la correction de défauts de vision. De manière particulièrement préférée, les implants oculaires faisant l'objet de la description sont des lentilles intra-cornéennes pour la correction de la myopie, de l'hypermétropie, de la presbytie, du kératocône ou des ectasies post-LASIK (kératocône iatrogène). L'implantation dans la cornée d'un lenticule ayant une forme déterminée, la cornée étant par exemple découpée au laser femtoseconde, permet de remodeler de l'intérieur une cornée déformée par la maladie kératocônique.

Outre les dispositions qui précédent, la description comprend encore d'autres dispositions qui ressortiront du complément de description qui suit, qui se rapporte à des exemples mettant en évidence les propriétés améliorées des objets en hydrogel biocompatible issus du procédé faisant l'objet de la description.

### EXEMPLE : Fabrication d'une lentille de contact en hydrogel de copolymère AN-69 selon le procédé faisant l'objet de la description

### Préparation d'un moule en hydrogel d'agarose :

Le moule en hydrogel d'agarose est préparé à partir d'une matrice en polypropylène (partie mâle et partie femelle) d'une lentille de contact, fournie par la Société Essilor.

Une solution aqueuse d'agarose à 2-4% est préparée en dissolvant de l'agarose dans du sérum physiologique (à 0,9% de NaCl dans H₂O) à une température de 80°C. Elle est ensuite refroidie à une température de 40-50°C, puis versée dans la matrice en polypropylène (dans les deux parties séparément).

Après refroidissement à température ambiante, on démoule les deux parties du moule en agarose obtenu. Les deux parties du moule sont ensuite immergées dans une solution physiologique (à 0,9% de NaCl dans H₂O).

### Préparation d'une solution polymérique :

Une solution polymérique répondant à la composition ci-après est préparée, sous agitation, et au bain-marie à une température de 60°C :

| Constituants | % en poids |
|---|---|
| Copolymère AN-69 (extrait sec) | 9 |
| Diméthylformamide (DMF) | 85 |
| Solution physiologique (à 0,9% de NaCl dans H₂O) | 6 |

### Fabrication d'une lentille de contact :

Une goutte de la solution polymérique préalablement préparée est versée dans la partie femelle du moule en hydrogel d'agarose précédemment préparé. La partie femelle du moule est immédiatement fermée par la partie mâle.

Après 30 secondes, le moule est ouvert. On procède alors à l'extraction de la forme gélifiée de la lentille de contact. Celle-ci est immergée deux fois successives pendant 30 minutes dans 0,5 L d'une solution physiologique à température ambiante, ce qui conduit à un échange total du DMF (solvant) par la solution physiologique (non solvant).

On obtient ainsi une lentille de contact de 10 mm de diamètre ayant une épaisseur centrale de 0,3-0,4 mm. Elle présente une puissance optique de 2,5 D et une contenance hydrique (tenue en eau) moyenne de 75%.

La lentille de contact est ensuite soumise à une stérilisation aux rayons Gamma. La dose de rayons gamma absorbée est de 25 Gray (ou 2,5 MRad).

Après stérilisation, la lentille de contact est placée dans une capsule contenant du sérum physiologique.

On constate les observations :
- la forme de la lentille (convexe/concave) n'a pas été modifiée,
- la contenance hydrique a diminué de 2%,
- la puissance optique a très faiblement variée (± 0,25 D).

## Revendications

1. Procédé de fabrication d'un objet en hydrogel biocompatible **caractérisé en ce qu'**il comprend les étapes suivantes :
(i) la préparation d'une solution polymérique par dissolution d'un copolymère d'acrylonitrile et d'un co-monomère oléfiniquement insaturé porteur de groupes anioniques dans un solvant aprotique, éventuellement en présence d'un non solvant,
(ii) la mise en forme et le début de la gélification de la solution polymérique obtenue à l'issu de l'étape (i) dans un moule constitué en un matériau contenant ledit non solvant ou en un matériau perméable audit non solvant,
(iii) l'immersion de l'objet en cours de gélification issu de l'étape (ii) dans un non solvant
dans lequel le non solvant est choisi parmi l'eau, les solutions aqueuses de sel minéral et les solutions aqueuses de sel organique, et
dans lequel le moule de l'étape (ii) est un moule en hydrogel.

2. Procédé selon la revendication 1 dans lequel le copolymère acrylonitrile/co-monomère présente un rapport molaire allant de 90/10 à 100/0, et de préférence allant de 95/5 à 99/1.

3. Procédé selon la revendication 1 ou la revendication 2 dans lequel les groupes anioniques du co-monomère oléfiniquement insaturé sont choisis parmi les groupes sulfonate, carboxylate, phosphate, phosphonate et sulfate.

4. Procédé selon l'une des revendications 1 à 3 dans lequel le copolymère acrylonitrile/co-monomère est un copolymère acrylonitrile-méthallylsulfonate de sodium.

5. Procédé selon l'une des revendications 1 à 4 dans lequel le solvant aprotique est choisi parmi les solvants aprotiques polaires organiques ou inorganiques.

6. Procédé selon la revendication 5 dans lequel le solvant aprotique est choisi parmi le diméthylformamide (DMF), le diméthylsulfoxyde (DMSO), la N,N-diméthylacétamide (DMAC), la N-méthylpyrrolidone (NMP).

7. Procédé selon l'une des revendications 1 à 6 dans lequel le non solvant est choisi parmi l'eau ou une solution aqueuse de chlorure de sodium.

8. Procédé selon l'une des revendications 1 à 7 dans lequel le moule de l'étape (ii) est un moule en hydrogel à base d'agarose, d'alginates, de polyhydroxyéthylméthacrylate (PHEMA), de polyhydroxypropylméthacrylate (PHPMA) ou de polyacrylate.

9. Procédé selon la revendication 8 dans lequel le moule de l'étape (ii) est un moule en hydrogel d'agarose.

10. Procédé selon la revendication 8 ou la revendication 9 dans lequel le moule de l'étape (ii) est constitué :
- de 1 à 10% en poids, et de préférence de 2 à 6%, d'agarose ou d'alginates, et
- de 90 à 99% en poids, et de préférence de 94 à 98%, d'eau ou d'une solution aqueuse de sel minéral ou d'une solution aqueuse de sel organique.

11. Procédé selon l'une des revendications 1 à 10 dans lequel l'étape (iii) d'immersion est réalisée en deux temps :
- dans un premier temps : immersion de l'objet en cours de gélification dans un bain froid de non solvant, de préférence à une température allant de 0 à 10°C, et
- dans un deuxième temps : immersion de l'objet en cours de gélification dans un bain de non solvant, à température ambiante.

12. Procédé selon l'une des revendications 1 à 11 **caractérisé en ce que** l'objet en hydrogel biocompatible fabriqué est choisi parmi les films, fils, joncs ou implants à usage médical, biologique, ophtalmologique et/ou extra-ophtalmologique.

13. Procédé selon la revendication 12 **caractérisé en ce que** l'objet en hydrogel biocompatible fabriqué est sous forme d'un implant oculaire.

14. Procédé selon la revendication 13, dans lequel lesdits implants oculaires sont des lentilles intra-cornéennes pour la correction de la myopie, de l'hypermétropie, de la presbytie, du kératocône ou des ectasies post-LASIK.

## Patentansprüche

1. Verfahren zur Herstellung eines Gegenstandes aus biokompatiblem Hydrogel, **dadurch gekennzeichnet, dass** es die folgenden Schritte umfasst:
(i) die Herstellung einer Polymerlösung durch Auflösen eines Acrylnitril-Copolymers und eines olefinisch ungesättigten Comonomers, das anionische Gruppen trägt, in einem aprotischen Lösungsmittel, eventuell bei Vorhandensein eines Nichtlösungsmittels,
(ii) die Formgebung und den Beginn des Gelierens der in Schritt (i) erhaltenen Polymerlösung in einer Form aus einem Material, das das Nichtlösungsmittel enthält, oder aus einem Material, das für das Nichtlösungsmittel durchlässig ist,
(iii) das Eintauchen des gelierenden Gegenstandes aus Schritt (ii) in ein Nichtlösungsmittel,
wobei das Nichtlösungsmittel ausgewählt ist aus Wasser, wässrigen anorganischen Salzlösungen und wässrigen organischen Salzlösungen, und
wobei die Form aus Schritt (ii) eine Hydrogel-Form ist.

2. Verfahren nach Anspruch 1, wobei das Acrylnitril-/Comonomer-Copolymer ein Molverhältnis von 90/10 bis 100/0, und vorzugsweise von 95/5 bis 99/1, aufweist.

3. Verfahren nach Anspruch 1 oder Anspruch 2, wobei die anionischen Gruppen des olefinisch ungesättigten Comonomers aus Sulfonat-, Carboxylat-, Phosphat-, Phosphonat- und Sulfatgruppen ausgewählt sind.

4. Verfahren nach einem der Ansprüche 1 bis 3, wobei das Acrylnitril-/Comonomer-Copolymer ein Acrylnitril-Natriummethallylsulfonat-Copolymer ist.

5. Verfahren nach einem der Ansprüche 1 bis 4, wobei das aprotische Lösungsmittel aus organischen oder anorganischen polaren aprotischen Lösungsmitteln ausgewählt ist.

6. Verfahren nach Anspruch 5, wobei das aprotische Lösungsmittel aus Dimethylformamid (DMF), Dimethylsulfoxid (DMSO), N,N-Dimethylacetamid (DMAC), N-Methylpyrrolidon (NMP) ausgewählt ist.

7. Verfahren nach einem der Ansprüche 1 bis 6, wobei das Nichtlösungsmittel aus Wasser oder einer wässrigen Natriumchloridlösung ausgewählt ist.

8. Verfahren nach einem der Ansprüche 1 bis 7, wobei die Form aus Schritt (ii) eine Hydrogel-Form basierend auf Agarose, Alginaten, Polyhydroxyethylmethacrylat (PHEMA), Polyhydroxypropylmethacrylat (PHPMA) oder Polyacrylat ist.

9. Verfahren nach Anspruch 8, wobei die Form aus Schritt (ii) eine Form aus Agarose-Hydrogel ist.

10. Verfahren nach Anspruch 8 oder Anspruch 9, wobei die Form aus Schritt (ii) gebildet wird:
- aus 1 bis 10 Gew.-%, vorzugsweise aus 2 bis 6 Gew.-%, Agarose oder Alginaten, und
- aus 90 bis 99 Gew.-%, vorzugsweise aus 94 bis 98 Gew.-%, Wasser oder einer wässrigen anorganischen Salzlösung oder einer wässrigen organischen Salzlösung.

11. Verfahren nach einem der Ansprüche 1 bis 10, wobei der Schritt des Eintauchens (iii) in zwei Phasen durchgeführt wird:
- in einer ersten Phase: Eintauchen des gelierenden Gegenstandes in ein kaltes Nichtlösungsmittelbad, vorzugsweise bei einer Temperatur von 0 bis 10°C, und
- in einer zweiten Phase: Eintauchen des gelierenden Gegenstandes in ein Nichtlösungsmittelbad bei Raumtemperatur.

12. Verfahren nach einem der Ansprüche 1 bis 11, **dadurch gekennzeichnet, dass** der hergestellte Gegenstand aus biokompatiblem Hydrogel ausgewählt wird aus Folien, Fäden, Stäben oder Implantaten zur medizinischen, biologischen, ophthalmologischen und/oder extra-ophthalmologischen Verwendung.

13. Verfahren nach Anspruch 12, **dadurch gekennzeichnet, dass** der hergestellte Gegenstand aus biokompatiblem Hydrogel die Form eines Augenimplantats hat.

14. Verfahren nach Anspruch 13, wobei die Augenimplantate intrakorneale Linsen zur Korrektur von Myopie, Hyperopie, Presbyopie, Keratokonus oder Post-LASIK-Ektasie sind.

## Claims

1. Method for manufacturing a biocompatible hydrogel object, **characterised in that** it comprises the following steps:
(i) the preparation of a polymeric solution by dissolution of a copolymer of acrylonitrile and an olefinically unsaturated anionic group carrier comonomer in an aprotic solvent, optionally in the presence of a non-solvent,
(ii) the shaping and the starting of the gelation of the polymeric solution obtained from step (i) in a mould constituted of a material containing said non-solvent or of a material which is permeable to said non-solvent,
(iii) the immersion of the object during its gelation from step (ii) in a non-solvent,
wherein the non-solvent is selected from among water, mineral salt aqueous solutions and organic salt aqueous solutions, and
wherein the mould of step (ii) is a mould in hydrogel.

2. Method according to claim 1, wherein the acrylonitrile/comonomer copolymer presents a molar ratio going from 90/10 to 100/0, and preferably going from 95/5 to 99/1.

3. Method according to claim 1 or claim 2, wherein the anionic groups of the olefinically unsaturated comonomer are selected from among the sulfonate, carboxylate, phosphate, phosphonate and sulphate groups.

4. Method according to one of claims 1 to 3, wherein the acrylonitrile/comonomer copolymer is a acrylonitrile- sodium methallylsulfonate copolymer.

5. Method according to one of claims 1 to 4, wherein the aprotic solvent is selected from among organic or inorganic polar aprotic solvents.

6. Method according to claim 5, wherein the aprotic solvent is selected from among dimethylformamide (DMF), dimethyl sulfoxide (DMSO), N,N-dimethylacetamide (DMAC), N-methyl pyrrolidone (NMP).

7. Method according to one of claims 1 to 6, wherein the non-solvent is selected from among water or a sodium chloride aqueous solution.

8. Method according to one of claims 1 to 7, wherein the mould of step (ii) is mould in agarose, alginate, polyhydroxyethylmethacrylate (PHEMA), polyhydroxypropylmethacrylate (PHPMA) or polyacrylate-based hydrogel.

9. Method according to claim 8, wherein the mould of step (ii) is mould in agarose hydrogel.

10. Method according to claim 8 or claim 9, wherein the mould of step (ii) is constituted:
- of 1 to 10% by weight, and preferably 2 to 6%, of agarose or of alginates, and
- of 90 to 99% by weight, and preferably 94 to 98%, of water or of a mineral salt aqueous solution or of an organic salt aqueous solution.

11. Method according to one of claims 1 to 10, wherein the immersion step (iii) is carried out in two stages:
- in a first stage: immersion of the object during its gelation in a cold bath of non-solvent, preferably at a temperature going from 0 to 10°C, and
- in a second stage: immersion of the object during its gelation in a bath of non-solvent, at ambient temperature.

12. Method according to one of claims 1 to 11, **characterised in that** the biocompatible hydrogel object manufactured is selected from among films, wires, rushes or implants for medical, biological, ophthalmological and/or extra-ophthalmological use.

13. Method according to claim 12, **characterised in that** the biocompatible hydrogel object manufactured is in the form of an ocular implant.

14. Method according to claim 13, wherein said ocular implants are intra-cornea lenses for the correction of myopia, hypermetropia, presbyopia, keratoconus or post-LASIK ectasias.
